# EUROPÄISCHE PATENTANMELDUNG

(11) **EP 1 586 555 A2**
(43) Veröffentlichungstag der Anmeldung: **19.10.2005**
(21) Anmeldenummer: 05004753.9
(22) Anmeldetag: 04.03.2005
(51) Int. Cl.: C07C 227/44, C07C 227/40, C07C 229/24

(54) **Lagerstabile Iminodisuccinatlösungen**

(30) Priorität: 16.03.2004 DE 102004012873
(71) Anmelder: LANXESS Deutschland GmbH, 51369 Leverkusen (DE)
(72) Erfinder: Mitschker, Alfred, Dr., 51519 Odenthal (DE); Schmidt, Holger, Dr., 41539 Dormagen (DE); Moritz, Ralf Johann, Dr., 41469 Neuss (DE); Wagner, Paul, Dr., 40597 Düsseldorf (DE); Feller, Rolf, 40822 Mettmann (DE)

(57) **Zusammenfassung**

Die vorliegende Erfindung betrifft ein Verfahren zur Herstellung von lagerstabilen Iminodisuccinatlösungen indem man eine wässrige Lösung von Iminodisuccinat mit einem Ammoniakgehalt von mehr als 50 ppm durch Temperung bei 40 bis 20°C über 5 Stunden bis zu 300 Tagen und anschließender Destillation bei <120°C unterzieht.

## Beschreibung

Die vorliegende Erfindung betrifft ein Verfahren zur Herstellung von lagerstabilen Iminodisuccinatlösungen indem man eine wässrige Lösung von Iminodisuccinat mit einem Ammoniakgehalt von mehr als 50 ppm durch Temperung bei 40 bis 120°C über 5 Stunden bis zu 300 Tagen und anschließender Destillation bei <120°C unterzieht.

Die Herstellung von Iminodisuccinat oder Iminodibernsteinsäure und ihren Salzen gehört zum Stand der Technik.

Nach GB-A 130 6 331 erhält man Iminodisuccinat durch Reaktion der Edukte Maleinsäure und Ammoniak bei 60 bis 155°C.

Nach SU-A 0639863 erhält man Iminodisuccinat durch Umsetzung derselben Edukte in Anwesenheit von Alkalimetallen bei 110-130°C.

WO-A 98/45251 offenbart schließlich die Synthese von Iminodibernsteinsäurealkalisalzen durch Umsetzung von Maleinsäureanhydrid, Alkalimetallhydroxid, NH₃ und Wasser, wobei das Reaktionsgemisch mit weiterem Wasser und gegebenenfalls Methanol versetzt und destillativ bei 50 bis 170°C und 0,1 bis 50 bar von NH₃ befreit wird.

Nachteilig an den Verfahrensprozessen des Standes der Technik ist die Tatsache, dass verfahrensbedingt das Produkt erhebliche Mengen Ammoniak enthält. Selbst durch die apparativ aufwändige Destillation in WO-A 98/45251 lässt sich der Ammoniak nicht dauerhaft reduzieren, so dass der Einsatz von Iminodisuccinaten bisher nur beschränkt möglich war.

Gerade aber Anwendungen wie Textilreinigung, Haushaltspflege und Kosmetik erfordern ein lagerstabiles Iminodisuccinat frei von NH₃-Ausgasungen, die durch Zersetzung des Iminodisuccinats oder von NH₃-Produktionsrückständen herrühren.

Die Aufgabe der vorliegenden Erfindung bestand also darin, ein Verfahren zur Herstellung lagerstabiler Iminodisuccinatlösungen zu finden, das es ermöglicht Iminodisuccinat-Lösungen über einen langen Zeitraum hinweg zu lagern, ohne dass sich Ammoniak bildet und dadurch die Anwendung in den oben genannten Bereichen ermöglicht wird.

Überraschenderweise wurde nun gefunden, dass eine Iminodisuccinatlösung, die über mehrere Stunden erhitzt worden ist, anschließend deutlich weniger Ammoniak bildet, als eine entsprechende nicht getemperte Lösung. Gleichzeitig weist eine Iminodisuccinatlösung nach der Erhitzung einen höheren Gehalt an Iminodisuccinat auf, als vor der Erhitzung. Es ist dadurch möglich, ein Produkt von deutlich größerer Reinheit und besseren Geruchseigenschaften zu erhalten. Obwohl Iminodisuccinat temperaturempfindlich ist und eine Erhitzung der Lösung zu einer chemischen Zersetzung unter Ammoniakbildung führt, wurde im Rahmen der vorliegenden Erfindung gefunden, dass durch gezielt dosierte Wärmezufuhr eine höhere Produktqualität hinsichtlich der Stabilität der Iminodisuccinatlösung gegen eine weiter fortschreitende Zersetzung und Ammoniakbildung erreicht werden kann.

Die Lösung und somit Gegenstand der vorliegenden Erfindung ist ein Verfahren zur Herstellung einer lagerstabilen Lösung von Iminodisuccinat mit einem Ammoniakgehalt <50 ppm, dadurch gekennzeichnet, dass man eine Iminodisuccinatlösung mit erhöhtem Ammoniakgehalt über 5 Stunden bis zu 300 Tagen bei 40 bis 120°C tempert und anschließend einer Destillation bei Temperaturen <120°C unterzieht.

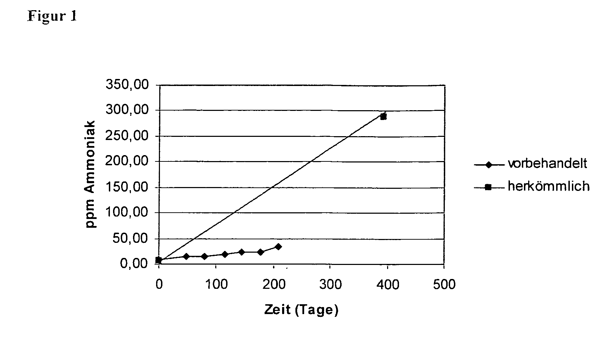

Tempern im Sinne der vorliegenden Erfindung bedeutet das Erhitzen eines Stoffes, hier einer Iminodisuccinat-Lösung mit erhöhtem Ammoniakgehalt, über einen längeren Zeitraum hinweg. Ein erhöhter Ammoniakgehalt im Sinne der vorliegenden Erfindung, und wie er durch die Iminodisuccinat-Synthesen des oben beschriebenen Standes der Technik im Produkt vorliegt, bedeutet 50 bis 500 ppm. Insbesondere durch Lagerung von Iminodisuccinatlösungen, wie sie gemäß den im Stand der Technik beschriebenen Verfahren erhalten werden, ist ein Anstieg der Ammoniak-Konzentration kontinuierlich auf Werte bis zu 150 ppm innerhalb von 200 Tagen zu beobachten (Fig. 1).

Eine erfindungsgemäß bei 90°C getemperte und anschließend einer Destillation unterzogene Iminodisuccinatlösung, hergestellt nach den oben genannten Verfahren des Standes der Technik, hat nach 200 Tagen nur 35 ppm Ammoniak gebildet (Fig. 1).

Der Befund, dass eine Temperung der Iminodisuccinatlösung zu besseren Produkteigenschaften führt, steht überraschenderweise im Gegensatz zu der Beobachtung, dass eine Iminodisuccinatlösung an Konzentration verliert, wenn sie erhitzt wird.

Iminodisuccinatlösungen im Sinne der vorliegenden Erfindung sind wässrige Lösungen von Iminodisuccinat, bevorzugt 0,1 bis 40 gew.-%ige wässrige Lösungen aber auch Mischungen dieser wässrigen Lösungen mit organischen Lösungsmitteln, beispielsweise Alkoholen. Als Alkohole seien beispielhaft Methanol, Ethanol, Propanole, Butanole genannt.

Die Erfindung betrifft bevorzugt ein Verfahren zur Herstellung lagerstabiler Lösungen von Iminodisuccinat mit einem Ammoniakgehalt < 50 ppm, dadurch gekennzeichnet, dass man eine Iminodisuccinat-Lösung mit erhöhtem Ammoniakgehalt 10 bis 100 Stunden, besonders bevorzugt 15 bis 70 Stunden bei 40 bis 120°C, besonders bevorzugt bei 80 bis 115°C, tempert und anschließend einer Destillation bei Temperaturen von 40 bis 130°C, besonders bevorzugt bei 60 bis 120°C unterzieht.

Man verfährt dabei in der Weise, dass man nach Herstellung der Iminodisuccinatlösung diese auf Temperaturen von 80 bis 120°C erwärmt und diese Temperaturen über 5 bis 200 h unter Durchmischung der Lösung aufrecht hält. Dies kann kontinuierlich oder diskontinuierlich geschehen. Im Falle der kontinuierlichen Verfahrensweise können geeignete Apparate ausgewählt und verschaltet werden, so dass die genannten Verweilzeiten bei den genannten Temperaturen erreicht werden. Hierzu sind in der chemischen und thermischen Verfahrenstechnik eine Reihe vorteilhafter Apparate und Apparateverschaltungen, wie z.B. die Kesselkaskade, der Rohrreaktor, Wärmeaustauscher mit Einbauten oder die kaskadierte Kolonne, bekannt.

In einem nachfolgenden Schritt erfolgt dann die Ammoniakdestillation kontinuierlich oder diskontinuierlich. Zur Durchführung dieses Destillationsschrittes sind dem Fachmann entsprechende Apparate und Destillationskonzepte bekannt.

Nach dieser Vorgehensweise können Iminodisuccinatlösungen erhalten werden, deren Ammoniakgehalt ohne weitere Nachbehandlung auch nach einer Zeitspanne von mehreren 100 Tagen 35 ppm nicht überschreitet (Fig. 1).

Gemäß dem erfindungsgemäßen Verfahren erhält man lagerstabile Iminodisuccinatlösungen mit einem Ammoniakgehalt unterhalb 50 ppm, bevorzugt unterhalb 40 ppm, besonders bevorzugt unterhalb 20 ppm, ja sogar unterhalb von 5 ppm!

Solch niedrige Ammoniak-Konzentration über einen Zeitraum von bis zu 200 Tagen erlauben erst den Einsatz dieser lagerstabilen Iminodisuccinatlösungen in Kosmetika, Waschmitteln, Reinigungsmitteln, in der Haushaltspflege, in der Papierherstellung, bei Bleichprozessen und in der Textilbehandlung.

### Beispiel

Nach Herstellung der Iminodisuccinatlösung (erhalten gemäßWO-A 98/45251) wurde diese im Reaktionskessel auf 102°C erwärmt und 20 Stunden unter Rühren bei dieser Temperatur gehalten. Anschließend wurden von der Lösung in einen Destillationskolonne bei Normaldruck Ammoniak und Wasser über Kopf abdestilliert. Hierbei ergaben sich Temperaturen im Sumpf von 105 bis 106°C. Im Kopf der Kolonne stellte sich eine Temperatur von 99°C ein.

Nach Versuchsende lag der Ammoniakgehalt in der destillierten Imminodisuccinatlösung unterhalb der Nachweisgrenze von 2 ppm. Nach 8 Wochen war der Ammoniakgehalt auf 16 ppm angestiegen.

## Patentansprüche

1. Verfahren zur Herstellung lagerstabiler Lösungen von Iminodisuccinat mit einem Ammoniakgehalt von <50 ppm, **dadurch gekennzeichnet, dass** man Iminodisuccinatlösungen mit einem Ammoniakgehalt über 50 ppm über 5 Stunden bis zu 300 Tagen bei 40 bis 120°C tempert und anschließend einer Destillation bei Temperaturen <120°C unterzieht.

2. Verfahren gemäß Anspruch 1, **dadurch gekennzeichnet, dass** die Temperung bei 80 bis 115 °C durchgeführt wird.

3. Verwendung der lagerstabilen Iminodisuccinatlösungen hergestellt nach Anspruch 1 für Kosmetika, Waschmittel, Reinigungsmittel, Haushaltspflege, in der Papierherstellung, bei Bleichprozessen und in der Textilbehandlung.
